# EUROPÄISCHE PATENTSCHRIFT

(11) **EP 0 994 120 B1**
(45) Veröffentlichungstag und Bekanntmachung des Hinweises auf die Patenterteilung: **17.07.2002**
(21) Anmeldenummer: 99250330.0
(22) Anmeldetag: 20.09.1999
(51) Int. Cl.: C07K 1/34, C07K 14/21, A61K 39/104

(54) **Verfahren zum Entfernen von Lipopolysacchariden aus wässrigen, proteinhaltigen Lösungen**
Process to remove lipopolysaccharides from aqueous solution of proteins
Procédé pour éliminer les lipopolysaccharides des solutions aqueuses de protéines

(30) Priorität: 06.10.1998 DE 19847074
(43) Veröffentlichungstag der Anmeldung: 19.04.2000
(73) Patentinhaber: Chiron Behring Gmbh And Co., 35041 Marburg (DE)
(72) Erfinder: Lenz, Uwe, 35083 Wetter (DE)
(74) Vertreter: UEXKÜLL & STOLBERG

(56) Entgegenhaltungen:
- EP-A- 0 088 303
- EP-A- 0 395 896
- GABELSBERGER J ET AL.: "A Hybrid Outer Membrane Protein Antigen for Vaccination Against Pseudomonas aeruginosa", BEHRING INSTITUT MITTEILUNGEN, , Februar 1997, Band 98, Nr. , Seiten 302 - 314

## Beschreibung

Die vorliegende Erfindung betrifft ein Verfahren zum Entfernen von Lipopolysacchariden (LPS) aus wäßrigen, proteinhaltigen Lösungen.

Erfindungsgemäß werden als wäßrige Lösungen jegliche Lösungen bezeichnet, die Wasser als Lösungsmittel enthalten.

Erfindungsgemäß werden als wäßrige, proteinhaltige Lösungen diejenigen wäßrigen Lösungen bezeichnet, die ein oder mehrere Proteine enthalten.

Lösungen und Geräte, die in der Medizin Verwendung finden, sind häufig mit Lipopolysacchariden kontaminiert. Die Ursache liegt in dem ubiquitären Vorkommen von Gram-negativen Bakterien, deren äußere Zellmembran LPS enthält. Kontaminationen mit LPS treten demgemäß häufig in Lösungen von Proteinen auf, die in Gram-negativen Bakterien rekombinant hergestellt wurden.

LPS bestehen aus drei unterschiedlichen Regionen, dem Lipid-A, dem Kern-Polysaccharid sowie der O-spezifischen Polysaccharidkette (N. Weary und F. Pearson, Biopharm (1988), 22-29; H. Schlegel, Allgemeine Mikrobiologie (1984), Thieme Verlag). In der äußeren Membran der Gram-negativen Bakterien spielen LPS eine Rolle als Permeabilitätsbarriere. Ferner tragen sie dort zur Stabilität der Zelle bei.

Lösungen, die zur Verwendung in Säugetieren vorgesehen sind, müssen frei von LPS sein, da LPS Fieber, Blutdruckabfall, Aggregation und Degeneration von Thrombozyten, intravasale Gerinnung durch Aktivierung von Faktor VII, Kachexie und septischen Schock hervorrufen (D.J. Hale et al. (1986), Handbook of Endotoxin, Vol. 4, pp. 1-17, Elsevier Science Publishing Co., Amsterdam; D.C. Morrison und D.M. Jacobs (1987), Ann.Rev.Med. 38 417-432; C.A. Janeway und P. Travers (1997), Immunologie, 2. Auflage, Spektrum Akademischer Verlag, 292-293). Auch bei in vitro-Behandlungen kann es erforderlich sein, die LPS zu entfernen, da beispielsweise Lymphozyten durch LPS unspezifisch stimuliert werden (M.P. Deutscher (1990), Methods in Enzymology (Guide to Protein Purification); Vol. 182, Academic Press, Inc., 317-328, 371-379).

Die möglichst vollständige Entfernung von LPS aus wäßrigen Lösungen stellt jedoch in der Regel ein schwerwiegendes Problem dar. Bei der Reinigung von rekombinant hergestellten Proteinen mittels dem Fachmann bekannter Methoden wie Gelfiltration oder Affinitätschromatographie werden zwar in aller Regel Begleitproteine abgetrennt, die LPS bleiben jedoch in Konzentrationen zurück, die die Toleranzgrenze für medizinische Anwendungen übersteigen. Unter Begleitproteinen werden im Sinne dieser Erfindung Proteine verstanden, die sich zusätzlich zu den gewünschten Proteinen in der wäßrigen Lösung befinden. Bei den Begleitproteinen kann es sich beispielsweise um bakterieneigene Proteine handeln.

Für die Entfernung von LPS aus wäßrigen, proteinhaltigen Lösungen sind bereits eine Reihe von chromatographischen und chemischen Methoden entwickelt worden (W. Fischer (1990), Eur. J. Biochem, 194, 655-661; T. Sugiyama (1990), Microb. Immun., 34, 635-641; R.S. Stinson, J. Biol. Standard; Y. Aida und M.J. Pabst (1900) J. Immun. Meth., 132, 191-195; S. Hjerten (1989), J. Chromatogr., 437, 237; I.M. Helander (1992), Molecular Microbio., 6, 2857-2862). Diese Methoden führen jedoch nicht zu einer ausreichenden Abtrennung von LPS, wenn die wäßrige, proteinhaltige Lösung nachfolgend beim Menschen oder bei Säugetieren eingesetzt werden soll.

EP 395 896 beschreibt ein Verfahren zur Abtrennung von Toxinen aus einer Proteinlösung, bei dem Toxine, wie beispielsweise bakterielle Lipopolysaccharide (LPS), in Gegenwart eines Chelatbildners und eines ionischen Detergenzes mittels einer Ionenaustauschchromatographie abgetrennt werden. Die Abtrennung erfolgt jedoch im basischen Milieu, zwischen pH7 und 10.

EP 088 303 beschreibt Verfahren zur Isolierung von Lipopolysacchariden aus Bakterien, bei denen man schrittweise quarternäres Ammoniumsalz, ein Salz eines Alkalimetalls oder Erdalkalimetalls, ein wasserlösliches Alkanol und schließlich ein Iodid, Thiocyanat oder ein Benzoat zugibt. Keine dieser Veröffentlichungen offenbart jedoch das Ansäuern einer wässrigen, proteinhaltigen Lösung zur Ausfällung der Lipopolysaccharide.

Die medizinisch akzeptable Toleranzgrenze liegt bei 25 EU/mg Gesamtprotein. EU (Endotoxin unit) beschreibt die biologische Aktivität eines Endotoxins. Eine EU ist als diejenige Aktivität definiert, die 100 pg des Standardtoxins EC-5 aufweisen.

Andere im Stand der Technik beschriebene Verfahren mittels toxikologisch bedenklicher Agenzien sind deswegen ungeeignet, weil nicht mit Sicherheit davon ausgegangen werden kann, daß nach Durchführung der Verfahren keine toxischen Substanzen in der Lösung verbleiben. Solche Verfahren betreffen den Einsatz von Polymyxinsäulen (K.W. Talmadge (1989, J. Chromatogr., 476, 175-85) oder die Anwendung der Phenol-Wasser-Extraktion nach Westphal (O. Westphal und K. Jann (1965), Methods Carbohydr. Chem., 5, 83-91). Ein weiteres Problem der Phenol-Wasser-Extraktion ist, daß dabei die in der wäßrigen Lösung vorhandenen Proteine denaturiert werden und dadurch ihre Wirksamkeit verlieren können.

Aufgabe der vorliegenden Erfindung ist es daher, ein Verfahren zur Verfügung zu stellen, bei dem man
1.) die Konzentrationen an Lipopolysacchariden in wäßrigen, proteinhaltigen Lösungen auf einen Wert herabsetzt, der unter der medizinischen Toleranzgrenze von 25 EU/mg Gesamtprotein liegt,
2.) der wäßrigen, proteinhaltigen Lösung keine Bestandteile zusetzt, die den Einsatz der gereinigten Lösung oder der Proteine in Säugetieren oder im Menschen aus toxikologischen Gründen bedenklich erscheinen lassen und
3.) die Proteine in der wäßrigen Lösung in ihrer nativen Konformation beläßt, da ein Denaturieren der Proteine ihre biologische Wirksamkeit herabsetzen kann.

Zur Lösung der gestellten Aufgabe wird ein Verfahren zum Entfernen von Lipopolysacchariden (LPS) aus wäßrigen, proteinhaltigen Lösungen vorgeschlagen, bei dem man die LPS durch Ansäuern der jeweiligen Lösung fällt und nachfolgend den Niederschlag von der wäßrigen, proteinhaltigen Lösung abtrennt.

Dem erfindungsgemäßen Verfahren liegt der überraschende Effekt zugrunde, daß LPS in einer wäßrigen, proteinhaltigen Lösung im sauren pH-Bereich ausfallen. Diese nicht erwartete Eigenschaft ermöglicht es, die Konzentration an LPS auf einen Wert herabzusetzen, der unter der medizinischen Toleranzgrenze von 25 EU/mg Gesamtprotein liegt. Gleichzeitig enthält die wäßrige, proteinhaltige Lösung nach der Durchführung des erfindungsgemäßen Verfahrens keine toxischen Substanzen, und die in der Lösung enthaltenen Proteine werden durch das erfindungsgemäße Verfahren nicht denaturiert. Ferner geht nur ein relativ geringer Anteil (< 40 %) der in der Lösung vorhandenen Proteine beim Entfernen der LPS verloren.

Erfindungsgemäß werden die LPS durch Ansäuern der wäßrigen, proteinhaltigen Lösung ausgefällt. Im Rahmen der Erfindung wird unter "Ansäuern der Lösung" verstanden, daß der pH-Wert der wäßrigen, proteinhaltige Lösung, aus der die LPS entfernt werden sollen, auf einen Wert kleiner als 6,5 eingestellt wird.

Gemäß einer bevorzugten Ausführungsform der Erfindung wird die Lösung durch Zugabe einer Säure angesäuert. Vorzugsweise hat die zugegebene Säure einen pH-Wert von 2,0 bis 5,0. Besonders bevorzugt hat sie einen pH-Wert von 2,5 bis 4,0, und ganz besonders bevorzugt einen pH-Wert von 3,0.

Nach einer besonders bevorzugten Ausführungsform ist die eingesetzte Säure verdünnte Phosphorsäure, die Verwendung anderer Säuren wie Salzsäure, Essigsäure oder Zitronensäure ist jedoch erfindungsgemäß eingeschlossen.

Gemäß einer bevorzugten Ausführungsform wird zum Ansäuern der pH-Wert der wäßrigen, proteinhaltigen Lösung auf einen Wert von 5,0 bis 6,4 eingestellt, gemäß einer besonders bevorzugten Ausführungsform auf einen Wert von 5,8 bis 6,2 und gemäß einer ganz besonders bevorzugten Ausführungsform auf einen Wert von 5,9 bis 6,0.

Im Anschluß an das Ansäuern der wäßrigen, proteinhaltigen Lösung wird diese gemäß einer bevorzugten Ausführungsform mindestens eine Stunde lang inkubiert, um die LPS möglichst quantitativ ausfallen zu lassen. Gemäß einer besonders bevorzugten Ausführungsform wird die Inkubation zwei Stunden lang durchgeführt.

Erfindungsgemäß wird die Inkubation der wäßrigen Lösung bei weniger als 10 °C durchgeführt. Bevorzugt wird die Inkubation bei weniger als 8 °C und besonders bevorzugt bei 4 °C durchgeführt.

Erfindungsgemäß wird der durch das Ansäuren der wäßrigen, proteinhaltigen Lösung auftretende Niederschlag abgetrennt. Der Niederschlag umfaßt ausgefallene LPS.

Der Verlust an Proteinen durch die Durchführung des erfindungsgemäßen Verfahrens ist geringer als 40 %, bezogen auf die vor Durchführung des erfindungsgemäßen Verfahrens vorhandene Menge.

Gemäß einer besonders bevorzugten Ausführungsform ist der Verlust an Proteinen kleiner als 30 %.

Die Abtrennung des Niederschlages von der wäßrigen, proteinhaltigen Lösung kann auf bekannte Weise wie beispielsweise Filtration, Zentrifugation oder Sedimentation erfolgen. Die Zentrifugation wird bevorzugt bei 4 °C und 600 x g 20 Minuten lang durchgeführt.

Gemäß einer bevorzugten Ausführungsform der Erfindung wird der Niederschlag durch einen oder mehrere Zentrifugationsschritte abgetrennt, gemäß einer besonders bevorzugten Ausführungsform durch einen oder mehrere Filtrationsschritte, gefolgt von einem oder mehreren Zentrifugationsschritten.

Durch das erfindungsgemäße Verfahren wird die Konzentration an LPS in der wäßrigen, proteinhaltigen Lösung auf einen Wert kleiner als 25 EU/mg Gesamtprotein herabgesetzt. Diese niedrige Konzentration an LPS ermöglicht den Einsatz der wäßrigen, proteinhaltigen Lösung in Säugetieren. Gemäß einer bevorzugten Ausführungsform der Erfindung wird die Konzentration an LPS auf einen Wert kleiner als 15 EU/mg Gesamtprotein herabgesetzt, gemäß einer besonders bevorzugten Ausführungsform auf einen Wert kleiner als 5 EU/mg Gesamtprotein.

Die LPS-Konzentration in der Lösung kann durch verschiedene Verfahren bestimmt werden. Besonders etabliert ist der Limulus-Test. Dieser Test beruht auf der Fähigkeit von LPS, die Gerinnungskaskade von Limulus polyphemus (Königskrabbe) zu aktivieren (R. Roth et al. (1989), J. Lab. Clin. Med., 114, 306-311). Ferner kann die LPS-Konzentration mittels SDS-Polyacrylamidgelelektrophorese und anschließendem Western-Blot bestimmt werden. Dazu wird ein Aliquot der wäßrigen proteinhaltigen Lösung mittels SDS-Polyacrylamidgelelektrophorese (U.K. Laemmli (1970), Nature, 227, 680-685) aufgetrennt und anschließend auf eine Nylon- oder Nitrocellulosemembran überführt (H. Towbin et al. (1979), Proc. Natl. Acad. Sci. USA, 76, 4350-4354). Danach können die LPS durch LPS-spezifische Antikörper detektiert werden. Ein weiteres Verfahren zur Bestimmung der LPS-Konzentration ist der dem Fachman bekannte Pyrogen-Test, bei dem die Induktion von Fieber in Kaninchen durch LPS gemessen wird.

Gemäß einer bevorzugten Ausführungsform der Erfindung wird die wäßrige, proteinhaltige Lösung vor dem Ansäuern auf einen pH-Wert größer oder gleich 6,5 eingestellt. In besonders bevorzugter Weise wird sie auf einen pH-Wert von 6,5 bis 8,0 eingestellt und ganz besonders bevorzugt ist ein pH-Wert von 6,5. Diese Einstellung kann durch die Zugabe einer Säure erfolgen. Erfindungsgemäß kann die Einstellung des pH-Wertes auch durch Umpuffern mittels Gelfiltration (G. von Jagov und H. Schägger (1994), A practical guide to membrane protein purification, Academic press, 31-34) vorgenommen werden. Weitere dem Fachmann bekannte Verfahren zum Einstellen des pH-Wertes sind Dialyse und Ultrafiltration.

Vorzugsweise wird die Proteinkonzentration in der wäßrigen, proteinhaltigen Lösung vor dem Ansäuern zum Ausfällen der LPS auf einen OD₂₈₀-Wert von 0,5 bis 2,0 eingestellt. In besonders bevorzugter Weise wird sie auf einen OD₂₈₀-Wert von 0,7 bis 1,1, und in ganz besonders bevorzugter Weise auf einen OD₂₈₀-Wert von 0,8 bis 1,0 eingestellt.

Gemäß einer besonderen Ausführungsform enthält die wäßrige, proteinhaltige Lösung, aus der die LPS entfernt werden sollen, Proteine, die in Gram-negativen Bakterien, vorzugsweise E. coli, rekombinant hergstellt wurden. Die rekombinante Herstellung von Proteinen ist im Stand der Technik bekannt (P. Balbas und F. Bolivar (1990), Methods Enzymol. 185, 14-37).

Wäßrige Lösungen, welche die rekombinant hergestellten Proteine umfassen, sind in der Regel durch Aufschluß der Bakterien, die sie hergestellt haben, erhältlich. In einigen Fällen ist es auch möglich, die Bakterien abzuzentrifugieren, falls die Bakterien die rekombinant hergestellten Proteine in das Medium abgeben.

Bevorzugt gewinnt man die wäßrige Lösung, die in Gram-negativen Bakterien hergestellte Proteine umfaßt, durch Aufschluß der Gramnegativen Bakterien. Die Bakterien können mit dem Fachmann bekannten Verfahren aufgeschlossen werden. Zu diesen Verfahren zählen Homogenisierung mit Hilfe einer French-Press, Aufschluß mit Hilfe von Ultraschall, osmotische Lyse oder einer Kugelmühle (M.P. Deutscher (1990), Methods in Enzymology (Guide to Protein Purification), Academic Press, Inc., Vol 182, 147 - 153), die Erfindung ist aber nicht auf diese beschränkt.

Besonders bevorzugt werden als Gram-negative Bakterien E. coli verwendet. Die Erfindung schließt die Reinigung von Lösungen rekombinanter Proteine aus anderen Gram-negativen Bakterien wie Listeria (z.B. L. monocytogenes, L. inocua), Salmonella (z.B. S. flagellin, S. typhimurium), Pseudomonas, Shigella und Klebsiella ein. Für die Herstellung rekombinanter Proteine werden vorzugsweise attenuierte Bakterienstämme verwendet.

Nach Aufschluß der Gram-negativen Bakterien können die LPS sofort mittels des erfindungsgemäßen Verfahrens abgetrennt werden. Erfindungsgemäß eingeschlossen ist auch, daß nach dem Aufschluß der Bakterien zunächst die in den Bakterien rekombinant hergestellten Proteine von Begleitproteinen befreit werden. Diese Abtrennung der Begleitproteine ist durch dem Fachmann bekannten Methoden möglich und hängt von der Natur der zu reinigenden Proteine ab. Zu den Methoden zählen vor allem Affinitätschromatographie, Gelfiltration, Dünnschichtchromatographie, präparative SDS-Polyacrylamidgelelektrophorese, Ionenaustauschchromatographie, hydrophobe Interaktionschromatographie (HIC), Fällung (z.B. durch Ammoniumsulfat, Trichloressigsäure), Ultrafiltration, (z.B. Rührzelle, Kasettensystem) und Zentrifugation (z.B. Ultrazentrifugation), die Erfindung ist aber nicht auf diese beschränkt.

Nach einer besonders bevorzugten Ausführungsform enthält die wäßrige, proteinhaltige Lösung das Hybridprotein OprF/I. Das Hybridprotein OprF/I umfaßt den C-terminalen Teil von OprF (Aminosäuren 190 bis 342), der an das reife OprI fusioniert ist. Beide Proteine sind Membranproteine von Pseudomonas aeruginosa (J. Gabelsberger und B. Knapp (1997), Behring Inst. Mitt., 98, 302-314). Zusätzlich umfaßt das Hybridprotein OprF/I am Aminoterminus sechs Histidine und eine Methionin-Alanin-Gruppe. Die Wirksamkeit dieses Proteins im Rahmen einer Vakzine gegen Nosokomialinfektionen konnte in einem Mausversuch nachgewiesen werden.

Nosokomialinfektionen sind Infektionen, die häufig durch Erreger verursacht werden, an denen der Mensch normalerweise nicht erkrankt. Übertragung der Erreger erfolgt gleichzeitig mit Behandlung und Pflege, die Krankheiten treten hauptsächlich in Krankenhäusern auf.

Gemäß einer weiteren bevorzugten Ausführungsform der Erfindung wird der pH-Wert der wäßrigen, proteinhaltigen Lösung im Anschluß an das Abtrennen des Niederschlages auf einen Wert größer oder gleich 6,5 eingestellt. Besonders bevorzugt ist ein Wert größer als 6,8, und ganz besonders bevorzugt ein Wert von 7,0 bis 7,5. Die Einstellung des pH-Wertes erfolgt erfindungsgemäß durch Zugabe einer Base oder durch Umpuffern mittels Gelfiltration. Geeignete Basen sind beispielsweise Natronlauge, Kaliumhydroxid, Tris(hydroxymethyl)-aminomethan (Tris), Natriumcarbonat, die Erfindung ist aber nicht auf diese beschränkt.

Vorzugsweise wird die Lösung nachfolgend sterilfiltriert. Dabei wird erfindungsgemäß ein Filter mit einem Porendurchmesser kleiner als 0,25 µm verwendet.

Nach einer bevorzugten Ausführungsform werden die Proteine von der wäßrigen, proteinhaltigen Lösung abgetrennt. Vorzugsweise geschieht dies im Anschluß an das Einstellen des pH-Wertes nach dem Abtrennen der LPS, besonders bevorzugt im Anschluß an das Sterilfiltrieren. Nach einer besonders bevorzugten Ausführungsform werden die Proteine von der wäßrigen, proteinhaltigen Lösung durch zumindest teilweises Entfernen des Wassers abgetrennt, vorzugsweise durch Lyophilisieren oder Fällen der Proteine. Nach einer ganz besonders bevorzugten Ausführungsform werden die Proteine in Pulverform überführt.

Gegenstand der Erfindung ist ferner ein Verfahren zum Herstellen einer von Lipopolysacchariden (LPS) freien Präparation rekombinanter Proteine aus Aufschlußmedien Gram-negativer Bakterien, bei dem man Begleitproteine der gewünschten Proteine aus dem Aufschlußmedium beseitigt und anschließend die LPS aus der wäßrigen, proteinhaltigen Lösung entfernt, das dadurch gekennzeichnet ist, daß man
a) den pH-Wert der von Begleitproteinen befreiten wäßrigen, proteinhaltigen Lösung auf einen Wert von 5,0 bis 6,4 einstellt,
b) zum Fällen der LPS die wäßrige, proteinhaltige Lösung bei weniger als 10 °C inkubiert und
c) den Niederschlag abtrennt.

Erfindungsgemäß bezeichnet der Ausdruck "Präparation rekombinanter Proteine" ein Gemisch, welches die in Gram-negativen Bakterien rekombinant hergestellten Proteine enthält. Dabei kann es sich um ein oder mehrere Proteine handeln. Vorzugsweise ist das Gemisch flüssig oder fest. Nach einer besonders bevorzugten Ausführungsform ist die Proteinpräparation entweder eine wäßrige Lösung, welche die rekombinant hergestellten Proteine enthält, oder ein Gemisch in Pulverform, das die rekombinant hergestellten Proteine enthält.

Erfindungsgemäß sind Aufschlußmedien wäßrige, proteinhaltige Lösungen, die durch Aufschluß Gram-negativer Bakterien erhalten werden. Dieser Aufschluß kann mittels dem Fachmann bekannten Methoden durchgeführt werden (siehe oben).

Die Begleitproteine können mittels dem Fachmann bekannten Methoden entfernt werden (siehe oben).

Das Abtrennen der LPS aus den Aufschlußmedien sowie das gemäß bevorzugter Ausführungsformen im Anschluß an Schritt c) durchgeführte Einstellen des pH-Wertes und Sterilfiltrieren wird wie bereits oben beschrieben durchgeführt. Durch das erfindungsgemäße Abtrennen der LPS wird die Konzentration an LPS in den Aufschlußmedien auf einen Wert kleiner als 25 EU/mg Gesamtprotein herabgesetzt.

Bevorzugt werden die Aufschlußmedien für das erfindungsgemäße Verfahren aus E. coli gewonnen. Die Erfindung schließt die Gewinnung der Aufschlußmedien aus anderen Gram-negativen Bakterien wie Listeria (z.B. L. monocytogenes, L. inocua), Salmonella (z.B. S. flagellin, S. typhimurium), Pseudomonas, Shigella und Klebsiella ein.

Nach einer besonders bevorzugten Ausführungsform wird durch das erfindungsgemäße Verfahren eine Proteinpräparation des Hybridproteins OprF/I hergestellt.

Gemäß einer bevorzugten Ausführungsform der Erfindung werden die rekombinanten Proteine von der wäßrigen, proteinhaltigen Lösung abgetrennt. Vorzugsweise geschieht dies im Anschluß an das Einstellen des pH-Wertes nach dem Abtrennen der LPS, besonders bevorzugt im Anschluß an das Sterilfiltrieren. Nach einer bevorzugten Ausführungsform werden die rekombinanten Proteine von der wäßrigen, proteinhaltigen Lösung durch zumindest teilweises Entfernen des Wassers abgetrennt, vorzugsweise durch Lyophilisieren oder Fällen der Proteine. Nach einer ganz besonders bevorzugten Ausführungsform werden die rekombinanten Proteine in eine pulverförmige Proteinpräparation überführt.

Gemäß einer bevorzugten Ausführungsform wird die Proteinpräparation zu einem Therapeutikum formuliert. Je nach Anwendungsgebiet werden der Proteinpräparation dem Fachmann bekannte, unterschiedliche, medizinisch verträgliche Trägerstoffe zugesetzt, die den Einsatz der Proteinpräparation als Therapeutikum ermöglichen (C.A. Janeway und P. Travers (1997), Immunologie, 2.Auflage, Spektrum Akademischer Verlag, 523-540). Außerdem wird bei der Formulierung zum Therapeutikum die Proteinkonzentration auf eine verträgliche und gleichzeitig wirksame Konzentration eingestellt. Diese Konzentration ist je nach Protein verschieden und kann mittels dem Fachmann bekannter Methoden ermittelt werden. Dazu zählen *in vitro* Versuche an Zellkulturen oder Tierversuche. Die Proteinkonzentration in wäßrigen Lösungen kann mitels dem Fachmann bekannter Methoden ermittelt werden (D.M. Kirschenbaum (1975), Anal. Biochem. 68, 46.5-484).

Besonders bevorzugt wird die Proteinpräparation zu einer Vakzine formuliert. Die Formulierung einer Vakzine wird unter GLP-Kriterien durchgeführt. Dabei sollten die eingesetzten Chemikalien beispielsweise nach DAB, PhEur, BP, Lebensmittelqualität spezifiziert sein. Desweiteren ist die Vakzine auf Sterilität, Endotoxingehalt und Stabilität hin zu überprüfen.

Wie oben erläutert beruht der wesentliche Vorteil der Erfindung darauf, daß LPS nahezu quantitativ aus wäßrigen, proteinhaltigen Lösungen abgetrennt werden können, ohne daß Proteine in wesentlichem Umfang verloren gehen. Die nach der erfindungsgemäßen Fällung der LPS erhaltenen wäßrigen, proteinhaltigen Lösungen können direkt bei Säugetieren oder beim Menschen eingesetzt werden, da zum Entfernen der LPS keine toxischen Substanzen eingesetzt werden müssen. Es ist ein weiterer Vorteil des erfindungsgemäßen Verfahrens, daß die gewünschten Proteine nicht denaturiert werden. Daher bleibt die native Konformation und damit die biologische Wirksamkeit der Proteine erhalten.

Im folgenden wird die Erfindung durch Figuren, Tabellen und Beispiele erläutert.

### Figurenlegende

### Figur 1

Schema für die Herstellung und Reinigung des Hybridproteins OprF/I aus *E. coli*

### Beispiele

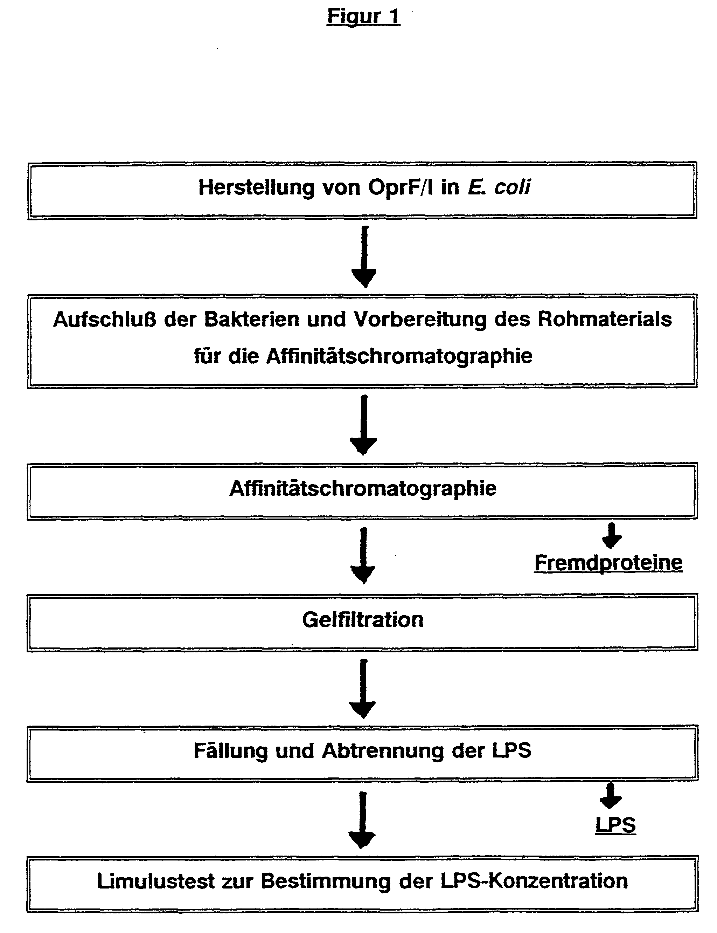

Zum Ablauf der Herstellung und Reinigung des Hybridproteins OprF/I siehe Figur 1.

### Beispiel 1

### Herstellung von rekombinantem OprF/I in Escherichia coli und Aufschluß der E. coli zur Gewinnung des Rohmaterials für die Chromatographie

Das für das OprF/I kodierende Gen wurde in den Vektor pTRC kloniert (J. Gabelsberger et al. (1997), Behring Inst. Mitt, 98, 302-314; E. Amann et al. (1988), Gene, 69, 301-315). *E. coli* XL1-Blue (Stratagene) wurden mit dem resultierenden Plasmid transformiert (J. Gabelsberger et al. (1997), Behring Inst. Mitt, 98, 302-314).

Die transformierten *E. coli* XL1-Blue werden in LB-Medium (1 % Trypton, o,5 % Hefeextrakt, 1 % NaCl) mit Ampicillin (100 µg/ml) bei 37 °C kultiviert.

Nach der Fermentation werden die rekombinanten Bakterien zentrifugiert (5000 g, 4 °C, 20 min). Das erhaltene Bakterienpellet wird in Phospaht-Puffer (0,1 M Natriumphosphat, 0,5 M NaCl, pH 8,0) suspendiert und mittels French-Press bei 700 bar aufgeschlossen.

Das durch den Aufschluß erhaltene Rohmaterial wird 1 Stunde lang bei ca. 48 000 x g zentrifugiert (Zentrifuge RC5C mit Rotor SS-34, Sorvall). Der erhaltene Überstand wird mittels Druckfiltration (Celluloseacetat-Filter, 0,2 µm, 142 mm, Sartorius SM 11107-142N; Gerät Edelstahlfiltrationsgerät, 142 mm, 200 ml, Sartorius (Bestellnr. SM 16274) bei 2,5 bar Überdruck filtriert.

Das Filtrat wird auf eine Imidazolkonzentration (Merck, Bestellnr. 104716) von 20 mM gebracht.

### Beispiel 2

### Affinitätschromatographie

Das OprF/I Hybridprotein umfaßt am N-terminalen Ende eine Gruppe aus 8 Aminosäuren, die sechs aufeinanderfolgende Histidine und jeweils ein Methionin und ein Alanin enthält. Dieses Protein kann somit über eine Säule, die Nickel enthält, aufgereinigt werden (S. Kanon (1986), A new support for the large scale purification of proteins, J. Chromatogr. 376, 259-267).

Das filtrierte Rohmaterial (Beispiel 1) wird mit ca. 40 cm/h auf die mit Äquilibrierungspuffer (0,10 M Natriumdihydrogenphosphat-1-hydrat, 0,50 M Natriumchlorid, 0,02 M Imidazol, pH 8,0, mit 1 M Natronlauge eingestellt) eingestellte Affinitätssäule (Gelmaterial: Ni-NTA Superflow® (Matrix: Sepharose® CL-6B, Ligand: Nitrilotriessigsäure (NTA)), Qiagen (Bestell-Nr. 30430); Chromatographiesäule: ECONO-Säule, Bio-Rad, d = 2,5 cm, V(Gel) = 22 mm) aufgetragen. Die Beladung der Säule beträgt ca. 9 ml Rohmaterial/ml Gel.

Nach dem Auftrag wird die Säule mit Äquilibrierungspuffer bei einer linearen Flußrate von ca. 50 bis 60 cm/h gespült. Dabei wird die optische Dichte des Durchflusses mit einem UV-Photometer bei 280 nm (Fa. Milton Roy Company, Spectronic 601) gemessen. Es wird solange gespült, bis die Grundlinie des Schreibers annähernd erreicht ist. Dann wird die Flußrate auf ca. 50 cm/h eingestellt.

Mit Waschpuffer (0,10 M Natriumdihydrogenphosphat-1-hydrat, 0,50 M Natriumchlorid, 0,05 M Imidazol, pH 8,0, eingestellt mit 1 M Natronlauge) werden die bei gleicher Flußrate und spezifisch gebundenen Fremdproteine abgetrennt. Die Abtrennung ist beendet, sobald die Grundlinie erreicht ist.

Die Elution des Produktes erfolgt bei 50 cm/h in einem Einstufenschritt durch den Elutionspuffer (0,10 M Natriumdihydrogenphosphat-1-hydrat, 0,50 M Natriumchlorid, 0,50 M Imidazol, pH 8,0, mit 1 M Natronlauge eingestellt). Die Fraktion wird anhand des Elutionsdiagramms gesammelt. Die gemessene UV-Absorption (280 nm) des Eluats sollte bei 2,0 liegen.

Die Affinitätssäule wird mit Milli Q-Wasser gespült und bis zum nächsten Lauf in 20%iger Ethanollösung gelagert.

Gegebenenfalls kann das Eluat, falls es nicht sofort weiterverarbeitet werden soll, über einen 0,2 µm Filter (Millex-GV, 0,22 µm, 28 mm Durchmesser, Millipore (Bestell-Nr. SLGV 025 BS)) filtriert werden.

### Beispiel 3

### Gelfiltration zur Einstellung des pH-Wertes und der Proteinkonzentration

Die Gelfiltration wird mit dem Gerät 2238 UVICORD SII, Pharmacia, durchgeführt. Das Eluat (Beispiel 2) wird auf eine mit Laufpuffer (0,02 M Natriumdihydrogenphosphat-1-hydrat, pH 6,5, eingestellt mit 1 M NaOH) äquilibrierte Gelfiltrationssäule (Gelmaterial: Sephadex G25 Medium, Pharmacia (Bestell-Nr. 17-0033-02); Chromatographiesäule XK50/30, Pharmacia, d = 5 cm, V(Gel) = 450 mm) aufgetragen. Das Auftragsvolumen beträgt < 10 % des Gelbettvolumens. Die Elution erfolgt mit dem Laufpuffer bei ca. 20 cm/h. Die optische Dichte des Eluats wird mit einem UV-Photometer bei 280 nm gemessen und durch einen Schreiber aufgezeichnet. Das Eluat wird anhand des Elutionsdiagramms fraktioniert und gesammelt.

Die Säule wird mit Milli-Q-Wasser gespült, mit 1,0 M NaOH über Nacht regeneriert und anschließend wieder mit Milli Q-Wasser gespült. Bei Standszeiten, die länger als 1 Woche sind, wird die Säule in 90%iger Ethanollösung aufbewahrt.

### Beispiel 4

### Fällung zur Abtrennung von LPS

Das Eluat der Gelfiltration (Beispiel 3) hat eine UV-Absorption von 0,8 bis 1,0. Der pH-Wert wird langsam unter Rühren bei Raumtemperatur mit Phosphatpuffer (0,02 M Natriumdihydrogenphosphat-1-hydrat, pH 3,0, eingestellt mit 1 : 2 verdünnter Phosphorsäure (Merck, Bestell-Nr. 573.1000)) auf einen pH-Wert zwischen 5,9 und 6,0 herabgesetzt. Nach einer Verweildauer im Ruhezustand von etwa 2 Stunden bei 4 °C im Kühlschrank wird der unlösliche Teil abfiltriert (Filter: Millex-GV, 0,22 µm, 28 mm Durchmesser, Millipore). Gegebenenfalls muß die Fällung 30 Minuten lang bei 6000 x g und 4°C abzentrifugiert werden.

Das Filtrat bzw. der Überstand wird mit 0,1 M NaOH auf pH 7,2 eingestellt.

Das so hergestellte Produkt wird über einen 0,2 µm Filter (Millipore) sterilfiltriert. Das Produkt wird bei -20 °C gelagert.

### Beispiel 4

### Limulustest zur Bestimmung der LPS-Konzentration

Der Limulustest zur Bestimmung der LPS-Konzentration wurde wie bei J.F. Cooper et al. (1971), J. Lab. Clin. Med., 78, 138-148 beschrieben durchgeführt. Dabei wurde die zu untersuchende Probe auf einen pH-Wert zwischen 6,5 und 7,5 eingestellt und dann das Amöbozyten-Lysat hinzugegeben. Trübung oder Gelbildung zeigt das Vorhandensein von LPS an.

Durch das erfindungsgemäße Verfahren konnte die LPS-Konzentration in der Proteinpräparation auf einen Wert unter 25 EU/mg Gesamtprotein herabgesetzt werden (Tabelle 1).

**Tabelle 1**

| wäßrige, OprF/I haltige Lösung | Volumen (V) ml | Extinktion (E) | Konzentration (C) mg/ml | Menge OprF/I mg | LPS EU/mg Gesamtprotein |
|---|---|---|---|---|---|
| vor der Fällung | 400 | 1,05 | 3,15 | 1256 | > 25000 |
| nach der Fällung | 740 | 0,40 | 1,2 | 888 | 25 |
| Ausbeute | | | | 70,7 % | |

Gezeigt sind die Resultate eines repräsentativen Versuches (TZ 288).

Die Extinktionswerte wurden durch Messungen mit einem UV-Photometer (Fa. Milton Roy Company, Spektonic 601) bei 280 nm erhalten.

Der LPS-Gehalt wurde mit dem Limulustest bestimmt.

## Patentansprüche

1. Verfahren zum Entfernen von Lipopolysacchariden (LPS) aus wäßrigen, proteinhaltigen Lösungen, **dadurch gekennzeichnet, daß** man die LPS durch Ansäuern der jeweiligen Lösung fällt und nachfolgend den Niederschlag von der wäßrigen, proteinhaltigen Lösung abtrennt.

2. Verfahren nach Anspruch 1, **dadurch gekennzeichnet, daß** man zum Ansäuern den pH-Wert der wäßrigen, proteinhaltigen Lösung auf einen Wert von 5,0 bis 6,4 einstellt.

3. Verfahren nach Anspruch 1 oder 2, **dadurch gekennzeichnet, daß** man zum Ansäuern den pH-Wert der wäßrigen, proteinhaltigen Lösung auf einen Wert von 5,8 bis 6,2 einstellt.

4. Verfahren nach den Ansprüchen 1 bis 3, **dadurch gekennzeichnet, daß** man zum Ansäuern den pH-Wert der wäßrigen, proteinhaltigen Lösung auf einen Wert von 5,9 bis 6,0 einstellt.

5. Verfahren zum Herstellen einer von Lipopolysacchariden (LPS) freien Präparation rekombinanter Proteine aus Aufschlußmedien Gram-negativer Bakterien, bei dem man Begleitproteine der gewünschten Proteine aus dem jeweiligen Aufschlußmedium beseitigt und anschließend die LPS aus der wäßrigen, proteinhaltigen Lösung entfernt, **dadurch gekennzeichnet, daß** man
a) den pH-Wert der von Begleitproteinen befreiten wäßrigen, proteinhaltigen Lösung auf einen Wert von 5,0 bis 6,4 einstellt,
b) zum Fällen der LPS die wäßrige, proteinhaltige Lösung bei weniger als 10 °C inkubiert, und
c) den Niederschlag abtrennt.

6. Verfahren nach den Ansprüchen 1 bis 5, **dadurch gekennzeichnet, daß** man vor dem Ansäuern der Lösung zum Fällen der LPS oder vor Schritt a) den pH-Wert der wäßrigen, proteinhaltigen Lösung auf einen Wert größer oder gleich 6,5 einstellt.

7. Verfahren nach den Ansprüchen 1 bis 6, **dadurch gekennzeichnet, daß** man vor dem Ansäuern der Lösung zum Fällen der LPS oder vor Schritt a) die Proteinkonzentration der wäßrigen, proteinhaltigen Lösung auf einen OD₂₈₀ Wert von 0,5 bis 2,0 einstellt.

8. Verfahren nach den Ansprüchen 1 bis 7, **dadurch gekennzeichnet, daß** man zur Fällung der LPS eine Säure mit einen pH-Wert von 2,0 bis 5,0 verwendet.

9. Verfahren nach den Ansprüchen 1 bis 8, **dadurch gekennzeichnet, daß** man als Säure Phosphorsäure verwendet.

10. Verfahren nach den Ansprüchen 1 bis 9, **dadurch gekennzeichnet, daß** man im Anschluß an das Ansäuern die Lösung bei weniger als 10 °C inkubiert.

11. Verfahren nach den Ansprüchen 1 bis 10, **dadurch gekennzeichnet, daß** man den Niederschlag durch Zentrifugieren abtrennt.

12. Verfahren nach den Ansprüchen 1 bis 11, **dadurch gekennzeichnet, daß** man die Konzentration der LPS in der wäßrigen, proteinhaltigen Lösung auf weniger als 25 EU/mg Gesamtprotein senkt.

13. Verfahren nach den Ansprüchen 1 bis 12, **dadurch gekennzeichnet, daß** die wäßrige, proteinhaltige Lösung in Gram-negativen Bakterien rekombinant hergestellte Proteine enthält.

14. Verfahren nach Anspruch 13, **dadurch gekennzeichnet, daß** man die wäßrige, proteinhaltige Lösung durch den Aufschluß der Gram-negativen Bakterien gewinnt.

15. Verfahren nach Anspruch 14, **dadurch gekennzeichnet, daß** man als Gram-negative Bakterien *E. coli* verwendet.

16. Verfahren nach den Ansprüchen 1 bis 15, **dadurch gekennzeichnet, daß** die wäßrige, proteinhaltige Lösung das Hybridprotein OprF/I enthält.

17. Verfahren nach den Ansprüchen 1 bis 16, **dadurch gekennzeichnet, daß** man den pH-Wert der wäßrigen, proteinhaltigen Lösung im Anschluß an das Abtrennen des Niederschlags von der Lösung auf einen Wert größer oder gleich 6,5 einstellt und die Lösung gegebenenfalls sterilfiltriert.

18. Verfahren nach den Ansprüchen 1 bis 17, **dadurch gekennzeichnet, daß** man die Proteine von der wäßrigen, proteinhaltigen Lösung abtrennt.

19. Verfahren nach den Ansprüchen 5 bis 18, **dadurch gekennzeichnet, daß** man die Proteinpräparation zu einem Therapeutikum formuliert.

20. Verfahren nach Anspruch 19, **dadurch gekennzeichnet, daß** man die Proteinpräparation zu einer Vakzine formuliert.

21. Verfahren zur Herstellung einer Vakzine, für die Vakzinierung gegen Nosokomialinfektionen, bei dem man ein Hybridprotein OprF/I gemäß einem der Ansprüche 5 bis 18 herstellt und zu einer Vakzine formuliert.

## Claims

1. A method for removing lipopolysaccharides (LPS) from aqueous, protein-containing solutions, wherein the LPS is precipitated by acidification of the respective solution, and the precipitate is subsequently separated from the aqueous, protein-containing solution.

2. The method of claim 1, wherein the pH of the aqueous, protein-containing solution is adjusted to 5.0 to 6.4 for acidification.

3. The method of claim 1 or 2, wherein the pH of the aqueous, protein-containing solution is adjusted to 5.8 to 6.2 for acidification.

4. The method of claims 1 to 3, wherein the pH of the aqueous, protein-containing solution is adjusted to 5.9 to 6.0 for acidification.

5. A method for producing a lipopolysaccharide (LPS)-free preparation of recombinant proteins from lysis media, of gram-negative bacteria, comprising the removal of proteins accompanying the desired proteins from the respective lysis medium and subsequent removal of LPS from the aqueous, protein-containing solution, wherein
a) the pH of the aqueous, protein-containing solution purified from accompanying proteins is adjusted to 5.0 to 6.4,
b) the aqueous, protein-containing solution is incubated at less than 10°C for precipitation of LPS, and
c) the precipitate is separated.

6. The method of claims 1 to 5, wherein the pH of the aqueous protein-containing solution is adjusted to 6.5 or higher before acidification of the solution for precipitation of LPS or before step a).

7. The method of claims 1 to 6, wherein the protein concentration of the aqueous, protein-containing solution is adjusted to an OD₂₈₀ of 0.5 to 2.0 before acidification of the solution for precipitation of LPS or prior to step a).

8. The method of claims 1 to 7, wherein an acid with a pH of 2.0 to 5.0 is used for precipitation of the LPS.

9. The method of claims 1 to 8, wherein the acid is phosphoric acid.

10. The method of claims 1 to 9, wherein the solution is incubated at less than 10°C after acidification.

11. The method of claims 1 to 10, wherein the precipitate is separated by centrifugation.

12. The method of claims 1 to 11, wherein the concentration of the LPS in the aqueous, protein-containing solution is reduced to less than 25 EU/mg of the total protein.

13. The method of claims 1 to 12, wherein the aqueous, protein-containing solution contains proteins, recombinantly produced in Gram-negative bacteria.

14. The method of claim 13, wherein the aqeous, protein-containing solution is obtained by the lysis of the Gram-negative bacteria.

15. The method of claim 14, wherein the Gram-negative bacteria are *E. coli.*

16. The method of claims 1 to 15, wherein the aqeuous, protein-containing solution contains the hybrid protein OprF/I.

17. The method of claims 1 to 16, wherein the pH of the aqueous, protein-containing solution is adjusted to a value which exceeds or corresponds to 6.5 after separation of the precipitate from the solution, and the solution is optionally sterilized by filtering.

18. The method of claims 1 to 17, wherein the proteins are separated from the aqueous, protein-containing solution.

19. The method of claims 5 to 18, wherein the protein preparation is formulated into a therapeutic composition.

20. The method of claim 19, wherein the protein preparation is formulated into a vaccine.

21. A method for the prepration of a vaccine for vaccination against nosocomial infections, wherein a hybrid protein OprF/I is prepared according to one of claims 5 to 18 and formulated into a vaccine.

## Revendications

1. Procédé pour éliminer les lipopolysaccharides (LPS) des solutions aqueuses de protéines, **caractérisé en ce que** l'on précipite les LPS par acidification de la solution concernée et **en ce que** l'on sépare ensuite le précipité de la solution aqueuse de protéines.

2. Procédé selon la revendication 1, **caractérisé en ce que**, pour l'acidification, on règle le pH de la solution aqueuse de protéines à une valeur comprise entre 5,0 et 6,4.

3. Procédé selon la revendication 1 ou 2, **caractérisé en ce que**, pour l'acidification, on règle le pH de la solution aqueuse de protéines à une valeur comprise entre 5,8 et 6,2.

4. Procédé selon les revendications 1 à 3, **caractérisé en ce que**, pour l'acidification, on règle le pH de la solution aqueuse de protéines à une valeur comprise entre 5,9 et 6,0.

5. Procédé pour la fabrication d'une préparation de protéines recombinantes exempte de lipopolysaccharides (LPS) à partir de milieux de dissolution de bactéries Gram-, au cours duquel on élimine les protéines d'accompagnement des protéines souhaitées du milieu de dissolution concerné puis on enlève les LPS de la solution aqueuse de protéines, **caractérisé en ce que** l'on
a) règle le pH de la solution aqueuse de protéines exempte de protéines d'accompagnement à une valeur comprise entre 5,0 et 6,4,
b) fait incuber la solution aqueuse de protéines à une température inférieure à 10°C pour précipiter les LPS, et
c) sépare le précipité.

6. Procédé selon les revendications 1 à 5, **caractérisé en ce que**, avant l'acidification de la solution pour la précipitation des LPS ou avant l'étape a), on règle le pH de la solution aqueuse de protéines à une valeur supérieure ou égale à 6,5.

7. Procédé selon les revendications 1 à 6, **caractérisé en ce que**, avant l'acidification de la solution pour la précipitation des LPS ou avant l'étape a), on règle la concentration en protéines de la solution aqueuse de protéines à une valeur OD₂₈₀ comprise entre 0,5 et 2,0.

8. Procédé selon les revendications 1 à 7, **caractérisé en ce que**, pour la précipitation des LPS, on utilise un acide ayant un pH compris entre 2,0 et 5,0.

9. Procédé selon les revendications 1 à 8, **caractérisé en ce que** l'on utilise de l'acide phosphorique comme acide.

10. Procédé selon les revendications 1 à 9, **caractérisé en ce que**, après l'acidification, on fait incuber la solution une température inférieure à 10°C.

11. Procédé selon les revendications 1 à 10, **caractérisé en ce que** l'on sépare le précipité par centrifugation.

12. Procédé selon les revendications 1 à 11, **caractérisé en ce que** l'on abaisse la concentration en LPS de la solution aqueuse de protéines en dessous de 25 EU/mg de protéine totale.

13. Procédé selon les revendications 1 à 12, **caractérisé en ce que** la solution aqueuse de protéines contient des protéines fabriquées par recombinaison dans des bactéries Gram-.

14. Procédé selon la revendication 13, **caractérisé en ce que** l'on obtient la solution aqueuse de protéines par la dissolution des bactéries Gram-.

15. Procédé selon la revendication 14, **caractérisé en ce que** l'on utilise *E. coli* comme bactéries Gram-.

16. Procédé selon les revendications 1 à 15, **caractérisé en ce que** la solution aqueuse de protéines contient la protéine hybride OprF/I.

17. Procédé selon les revendications 1 à 16, **caractérisé en ce que**, après la séparation du précipité de la solution, on règle le pH de la solution aqueuse de protéines à une valeur supérieure ou égale à 6,5 et on soumet éventuellement la solution à une filtration stérile.

18. Procédé selon les revendications 1 à 17, **caractérisé en ce que** l'on sépare les protéines de la solution aqueuse de protéines.

19. Procédé selon les revendications 5 à 18, **caractérisé en ce que** l'on formule la préparation de protéines sous la forme d'un médicament.

20. Procédé selon la revendication 19, **caractérisé en ce que** l'on formule la préparation de protéines sous la forme d'un vaccin.

21. Procédé pour la préparation d'un vaccin pour la vaccination contre les infections nosocomiales, dans lequel on prépare une protéine hybride OprF/I selon l'une des revendications 5 à 18 et on la formule sous forme d'un vaccin.
